# EUROPEAN PATENT APPLICATION

(11) **EP 3 904 372 A1**
(43) Date of publication of application: **03.11.2021**
(21) Application number: 19906177.1
(22) Date of filing: 26.12.2019
(51) Int. Cl.: C07K 14/62, C07K 1/18, B01D 15/36

(54) **INSULIN PRECURSOR PURIFYING METHOD USING ANION EXCHANGE CHROMATOGRAPHY**

(30) Priority: 27.12.2018 KR 20180170530
(71) Applicant: Polus Inc., Incheon 21984 (KR)
(72) Inventor: KIM, Jinhwan, Incheon 21988 (KR); BYUN, Seungmin, Bucheon-si Gyeonggi-do 14597 (KR); SONG, Jeyoun, Incheon 21997 (KR); KIM, Hyunuk, Incheon 21986 (KR); YOON, Jungpil, Sejong 30127 (KR)
(74) Representative: Budde Schou A/S
(86) International application number: PCT/KR2019/018468
(87) International publication number: WO 2020/138953

(57) **Abstract**

The present invention relates to an insulin precursor purifying method for improving the production yield of an insulin precursor by adjusting the pHs of a first buffer solution for equilibrating an ion exchange resin and a second buffer solution for eluting an insulin precursor bound to the ion exchange resins. The insulin precursor purifying method according to the present invention is a high-purity and high-yield insulin precursor purifying method for reinforcing by mean of suitable pH combination of buffer solutions, the binding force of an insulin precursor to ion exchange resins and enabling the insulin precursor to be effectively eluted thereafter, and is very useful in high-yield insulin production through enzymatic conversion after a purification process.

<110>    POLUS Inc.

<120>    A Method for Purifying Proinsulin Using Anion Exchange
         Chromatography

<130>    PP-B2348

<150>    KR 10-2018-0170530
<151>    2018-12-27

<160>    4

<170>    KoPatentIn 3.0

<210>    1
<211>    21
<212>    PRT
<213>    Artificial Sequence

<220>
<223>    Insulin A chain


<400>    1
Gly Ile Val Glu Gln Cys Cys Thr Ser Ile Cys Ser Leu Tyr Gln Leu
  1               5                  10                  15

Glu Asn Tyr Cys Asn
             20


<210>    2
<211>    30
<212>    PRT
<213>    Artificial Sequence

<220>
<223>    Insulin B chain


<400>    2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr
             20                  25                  30


<210>    3
<211>    21
<212>    PRT
<213>    Artificial Sequence

<220>
<223>    Insulin glargine A chain


<400>    3
Gly Ile Val Glu Gln Cys Cys Thr Ser Ile Cys Ser Leu Tyr Gln Leu
  1               5                  10                  15

Glu Asn Tyr Cys Gly
             20

<210>    4
<211>    32
<212>    PRT
<213>    Artificial Sequence

<220>
<223>    Insulin glargine B chain


<400>    4
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

## Description

### [Technical Field]

The present invention relates to a method for purifying an insulin precursor using anion exchange chromatography, and more particularly to a method for purifying an insulin precursor capable of improving the production yield of the insulin precursor by adjusting the pH of a first buffer solution for equilibrating an ion exchange resin and a second buffer solution for eluting the insulin precursor bound to the ion exchange resin.

### [Background Art]

Diabetes is a metabolic disease characterized by high blood sugar, and is caused by the complex action of genetic and environmental factors. Diabetes causes conditions such as type 1 diabetes, type 2 diabetes, gestational diabetes, hyperglycemia and the like, and is a metabolic disorder in which the pancreas produces an insufficient amount of insulin or in which cells of the human body do not respond properly to insulin, resulting in decreased ability to uptake glucose, and consequently, glucose accumulates in the blood.

The most representative method for the treatment of diabetes is a method for controlling a patient's blood sugar to a normal level by administering insulin. Insulin is a blood sugar control hormone secreted by the pancreas of the human body, and it plays a role in moving excess glucose in the blood to the cells so as to supply the cells with an energy source while maintaining blood sugar at a normal level.

With the development of technology for manufacturing a recombinant protein, various types of insulin products have been launched, and insulin products may be broadly classified into five types depending on the reactivity thereof. Specifically, rapid-acting insulin, which shows the fastest response, begins to act between 1 minute and 20 minutes due to the fast effect thereof, and exhibits the best effect after about 1 hour, and the effect thereof lasts for 3 to 5 hours, and representative examples thereof include insulin aspart (NovoRapid®) , insulin lispro (Humalog®), and insulin glulisine (Apidra®). The next-fastest-acting insulin is short-acting insulin, and short-acting insulin begins to lower blood sugar level about 30 minutes after administration and shows the best effect between 2 and 4 hours, and the effect thereof lasts for 6 to 8 hours. Representative examples of short-acting insulin include Actrapid®, Humilin®, Hypurin Neutral, and the like. Intermediate-acting insulin, containing protamine or zinc to prolong the action of insulin, begins to act about 1 hour and 30 minutes after injection, and the effect thereof reaches the maximum level between 4 and 12 hours and lasts for 16 to 24 hours. Representative examples thereof include Protaphane®, Humulin® NPH, and Hypurin Isophane®. Mixed insulin is a pre-mixed combination of rapid-acting insulin or short-acting insulin with intermediate-acting insulin so that two types of insulin may be easily administered through a single injection, and NovoMix® 30 (30% insulin aspart, 70% protamine crystallized insulin aspart), Humalog® Mix 25 (25% insulin lispro, 75% insulin lispro protamine suspension), and Humalog® Mix 50 (50% insulin lispro, 50% insulin lispro protamine suspension) are commercially available. Long-acting insulin is an insulin, which is injected once or twice a day and in which the effect thereof lasts up to 24 hours, and is usually used as a basal insulin, and Lantus® (insulin glargine, EP 0368187), Levemir® (insulin detemir, US 5,750,497), and Tresiba® (insulin degludec, US 7,615,532) are marketed.

Meanwhile, insulin is subjected to various post-translational modifications depending on the production pathway thereof. Production and secretion thereof are independent, and produced insulin is stored for secretion. C-peptide and mature insulin exhibit biological activity.

In mammals, insulin is synthesized in the beta cells of the pancreas, and insulin is composed of two polypeptide chains, namely an A-chain and a B-chain, which are linked by disulfide bonds. Early insulin is synthesized into a single polypeptide called preproinsulin in beta cells. Preproinsulin contains a signal peptide of 24 amino acid residues that moves new polypeptide chains into the rough endoplasmic reticulum. The signal peptide induces movement into the lumen of the rough endoplasmic reticulum, followed by cleavage to form proinsulin. In the rough endoplasmic reticulum, proinsulin folds into the correct shape and forms three disulfide bonds. 5-10 minutes after assembly in the endoplasmic reticulum, proinsulin is transported into the trans-Golgi network, where immature granules are formed.

Proinsulin matures into active insulin by the activity of exoprotease carboxypeptidase E and cellular endopeptidases known as prohormone convertases (PC1, PC2). Endopeptidase induces cleavage at two positions to release a fragment called C-peptide, and two peptide chains, namely a B-chain and an A-chain, are linked by two disulfide bonds. Each cleavage site is located after a pair of basic residues (lysine (Lys)-64 and arginine (Arg)-65, and arginine (Arg)-31 and arginine (Arg)-32). After the C-peptide is cleaved, these two pairs of basic residues are removed by carboxypeptidase. C-peptide is located in the central portion of proinsulin, and the primary structure of proinsulin corresponds to "B-C-A" in that order (the B-chain and the A-chain were identified based on mass, and the C-peptide was later discovered).

The produced mature insulin (active insulin) is packaged in mature granules, and is secreted from the cells into the circulatory system by metabolic signals (e.g., leucine (Leu), arginine (Arg), glucose, mannose) and vagus nerve stimulation.

In order to treat diabetes, active insulin is administered. With regard to a gene-recombinant insulin production technology for producing active insulin, Eli Lilly and Company used a method in which the A-chain and the B-chain are expressed using *E. coli* and mixed *in vitro* to form a disulfide bond and the A- and B-chains are linked, but there is a problem in that the production efficiency is not good. Eli Lilly and Company subsequently devised a method of producing insulin by expressing proinsulin, forming a disulfide bond *in vitro,* and cleaving C-peptide with trypsin and carboxypeptidase B.

Novo Nordisk Inc. developed a method of obtaining insulin by expressing, in yeast, mini-proinsulin in which B- and A-chains are linked by two basic amino acids, followed by trypsinization under laboratory conditions. This method has the advantage of formation of a disulfide bond during expression and secretion of mini-proinsulin and of easy separation and purification due to secretion in the medium, but it is difficult to produce on as large a scale as when using *E. coli.*

The development of novel gene-recombinant insulin production methods has been thoroughly carried out since then. Hoechst AG developed a method of obtaining insulin in which a novel insulin derivative or preproinsulin is expressed in *E. coli* and a disulfide bond is formed under *in-vitro* conditions, followed by treatment with lysyl endopeptidase or clostripain and carboxypeptidase B, and Bio-Technology General Corporation improved the expression effect and the disulfide bond formation efficiency under *in-vitro* conditions by expressing a fusion protein in which proinsulin is linked to superoxide dismutase (SOD) in *E. coli.* Conversion into insulin was performed with trypsin and carboxypeptidase B. As described above, many attempts have been made to realize gene-recombinant insulin production methods, and improvements have been made in view of the expression efficiency, disulfide bond formation efficiency, and method of conversion into insulin (KR 10-2001-7013921).

The present inventors have ascertained that, in order to increase the purity and yield of insulin glargine in the process of enzymatic conversion of an insulin glargine precursor, having improved persistence due to the increased *in-vivo* half-life thereof compared to native insulin, into insulin glargine, a purification process is required after inducing refolding of an insulin glargine precursor, and in particular, when the pH of the buffer solution and the concentration of the salt in the equilibration step of the ion exchange resin and the elution step in the anion exchange chromatography used for the purification process are appropriately adjusted, the purity and yield of insulin glargine that is subsequently produced may be notably increased, thus culminating in the present invention.

### [Disclosure]

It is an object of the present invention to provide a method for purifying an insulin precursor with high yield and high purity.

In order to achieve the above and other objects, the present invention provides a method for purifying an insulin precursor comprising:
(a) equilibrating an anion exchange resin with a first buffer solution;
(b) introducing a solution comprising an insulin precursor into the equilibrated anion exchange resin; and
(c) eluting the insulin precursor bound to the anion exchange resin using a second buffer solution having a pH higher than that of the first buffer solution.

### [Mode for Invention]

Unless otherwise defined, all technical and scientific terms used herein have the same meanings as those typically understood by those skilled in the art to which the present invention belongs. Generally, the nomenclature used herein and the test method described below are well known in the art and are typical.

In the present invention, when the pH of a buffer solution used in an elution step is adjusted to be higher than the pH of a buffer solution used for equilibration of an ion exchange resin, unlike conventional anion exchange chromatography in a purification process for effectively removing impurities generated during a series of processes of inducing refolding of insulin produced through genetic recombination technology, it is confirmed that an insulin precursor may be purified with high purity/high yield.

Accordingly, an aspect of the present invention pertains to a method for purifying an insulin precursor comprising:

(a) equilibrating an anion exchange resin with a first buffer solution;

(b) introducing a solution comprising an insulin precursor into the equilibrated anion exchange resin; and

(c) eluting the insulin precursor bound to the anion exchange resin using a second buffer solution having a pH higher than that of the first buffer solution.

As used herein, the term "insulin precursor" refers to a single-stranded peptide comprising an insulin A-chain and an insulin B-chain, with a C-peptide therebetween, and may be used interchangeably with "proinsulin". In the present invention, the insulin precursor conceptually comprises all precursor forms such as native insulin precursors, insulin analogue precursors, and derivatives thereof. The insulin precursor may be prepared by those of ordinary skill in the art with reference to methods disclosed in documents such as EP 0,211,299, EP 0, 227, 938, EP 0, 229, 998, EP 0,286,956, or KR 10-0158197.

As used herein, the term "insulin" refers to a protein that controls blood sugar in the body. Native insulin is a hormone secreted by the pancreas, and typically promotes intracellular glucose uptake and inhibits the breakdown of fat, and thus plays a role in controlling blood sugar in the body. In the present invention, insulin conceptually comprises all forms such as native insulin, insulin analogues, and derivatives thereof.

For insulin, an insulin precursor (proinsulin) having no blood sugar control function is processed into insulin having a blood sugar control function. Insulin is composed of two polypeptide chains, particularly an A-chain and a B-chain, each comprising 21 and 30 amino acid residues, which are linked by two disulfide bridges. The A-chain and B-chain of native insulin may comprise the following amino acid sequences.

A-chain: 
Gly-Ile-Val-Glu-Gln-Cys-Cys-Thr-Ser-Ile-Cys-Ser-Leu-Tyr-

Gln-Leu-Glu-Asn-Tyr-Cys-Asn (SEQ ID NO: 1)
B-chain:

Phe-Val-Asn-Gln-His-Leu-Cys-Gly-Ser-His-Leu-Val-Glu-Ala-

Leu-Tyr-Leu-Val-Cys-Gly-Glu-Arg-Gly-Phe-Phe-Tyr-Thr-Pro-

Lys-Thr (SEQ ID NO: 2)

The insulin precursor and insulin used in the present invention may be of human origin, but the present invention is not limited thereto.

In the present invention, the insulin analogue comprises one in which the amino acid of the B-chain or the A-chain is mutated compared to the native type. The *in-vivo* blood sugar control function of the insulin analogue may be the same as or may correspond to that of native insulin. Specifically, the insulin analogue precursor or insulin analogue may be configured such that at least one amino acid of native insulin is subjected to any variation selected from the group consisting of substitution, addition, deletion, modification, and combinations thereof, but the present invention is not limited thereto.

The insulin analogue that may be used in the present invention comprises an insulin analogue made by genetic recombination technology, and the insulin analogue conceptually comprises inverted insulin, insulin variants, insulin fragments, and the like.

The derivative has a blood sugar control function in the body, exhibits homology to each of the amino acid sequences of the A-chain and B-chain of the native insulin or insulin analogue, and comprises a peptide in a form in which some groups of one amino acid residue are chemically substituted (e.g. alpha-methylation, alpha-hydroxylation), removed (e.g. deamination), or modified (e.g. N-methylation). The insulin fragment is a form in which at least one amino acid is added to or deleted from insulin, and the added amino acid may be an amino acid that does not exist in nature (e.g. a D-type amino acid), and such an insulin fragment plays a blood sugar control function in the body.

The insulin variant is a peptide having a sequence in which at least one amino acid is different from that of insulin, and plays a blood sugar control function in the body.

The insulin analogue, derivative, fragment and variant of the present invention may be used independently or in combination. For example, a peptide, which has a sequence in which at least one amino acid is different, in which the amino-terminal amino acid residue is subjected to deamination, and which plays a blood sugar control function in the body, is also comprised in the scope of the present invention.

Specifically, in the present invention, the insulin analogue may be insulin glargine. Insulin glargine is stabilized by substituting asparagine, which is the 21^{st} amino acid of the A-chain of insulin, with glycine, and is also made soluble at a weakly acidic pH by adding two arginines to the carboxy terminus of the B-chain. Here, insulin glargine is an insulin analogue developed such that it forms a microprecipitate in subcutaneous tissue when administered with an acidic solution (pH 4.0) and is slowly dissolved and released from the microprecipitate, which is an insulin glargine hexamer, whereby the action time is prolonged up to 24 hours. The A-chain and B-chain of insulin glargine may comprise the following amino acid sequences (US 5, 656, 722).

A-chain: 
Gly-Ile-Val-Glu-Gln-Cys-Cys-Thr-Ser-Ile-Cys-Ser-Leu-Tyr-

Gln-Leu-Glu-Asn-Tyr-Cys-Gly (SEQ ID NO:3)
B-chain:

Phe-Val-Asn-Gln-His-Leu-Cys-Gly-Ser-His-Leu-Val-Glu-Ala-
Leu-Tyr-Leu-Val-Cys-Gly-Glu-Arg-Gly-Phe-Phe-Tyr-Thr-Pro-
Lys-Thr-Arg-Arg (SEQ ID NO: 4)

In the present invention, the anion exchange resin for use in anion exchange chromatography may be a diethylaminoethyl cellulose-based resin, but is not limited thereto.

Specifically, the anion exchange resin may be Fractogel EMD DEAE or Capto DEAE, but is not limited thereto.

The equilibration conditions of the ion exchange resin that is used for chromatography may be as follows: the pH of the first buffer solution may fall in the range of 7.0 to 8.0, preferably 7.8 to 8.0, and most preferably 7.9, but is not limited thereto, and also, the first buffer solution may be 10-100 mM Tris-HCl or borate, and preferably 20-50 mM Tris-HCl or borate, but is not limited thereto.

The elution conditions for chromatography may be as follows: the pH of the second buffer solution may fall in the range of 8.0 to 10.0, preferably 9.0 to 9.4, and more preferably 9.2, but is not limited thereto, and also, the second buffer solution may be 10-100 mM Tris-HCl or borate containing 0-200 mM sodium chloride, but is not limited thereto. The second buffer solution that may be used in the present invention is preferably selected from the group consisting of (i) 50 mM Tris-HCl at a pH of 8.0 to 9.0 containing 0-100 mM sodium chloride (NaCl), (ii) 50 mM borate at a pH of 9.2 containing 10 mM sodium chloride (NaCl), (iii) 50 mM borate at a pH of 9.4 containing 30 mM sodium chloride (NaCl), and (iv) 50 mM Tris-HCl at a pH of 8.0 to 9.0 containing 150-200 mM sodium chloride (NaCl), but is not limited thereto.

In an embodiment of the present invention, the insulin precursor may be purified using the method comprising (a) equilibrating Fractogel EMD DEAE with 20 mM Tris-HCl at a pH of 7.8 to 7.9, (b) introducing a refolding solution comprising an insulin precursor into the equilibrated Fractogel EMD DEAE, and (c) eluting the bound insulin precursor with 50 mM Tris-HCl at a pH of 8.0 to 9.0 containing 50-100 mM sodium chloride (NaCl).

In another embodiment of the present invention, the insulin precursor may be purified using the method comprising (a) equilibrating Fractogel EMD DEAE with 20 mM Tris-HCl at a pH of 7.8 to 7.9, (b) introducing a refolding solution comprising an insulin precursor into the equilibrated Fractogel EMD DEAE, and (c) eluting the bound insulin precursor with 50 mM borate at a pH of 9.2 containing 10 mM sodium chloride (NaCl).

In still another embodiment of the present invention, the insulin precursor may be purified using the method comprising (a) equilibrating Fractogel EMD DEAE with 20 mM Tris-HCl at a pH of 7.8 to 7.9, (b) introducing a refolding solution comprising an insulin precursor into the equilibrated Fractogel EMD DEAE, and (c) eluting the bound insulin precursor with 50 mM borate at a pH of 9.4 containing 30 mM sodium chloride (NaCl).

In yet another embodiment of the present invention, the insulin precursor may be purified using the method comprising (a) equilibrating Fractogel EMD DEAE with 50 mM Tris-HCl at a pH of 8.0, (b) introducing a refolding solution comprising an insulin precursor into the equilibrated Fractogel EMD DEAE, and (c) eluting the bound insulin precursor with 50 mM Tris-HCl at a pH of 9.2.

In still yet another embodiment of the present invention, the insulin precursor may be purified using the method comprising (a) equilibrating Capto DEAE with 20 mM Tris-HCl at a pH of 7.8 to 7.9, (b) introducing a refolding solution comprising an insulin precursor into the equilibrated Capto DEAE, and (c) eluting the bound insulin precursor with 50 mM Tris-HCl at a pH of 8.0 to 9.0 containing 150-200 mM sodium chloride (NaCl).

A better understanding of the present invention may be obtained through the following examples. These examples are merely set forth to illustrate the present invention, and are not to be construed as limiting the scope of the present invention, as will be obvious to those of ordinary skill in the art.

### Example 1. Induction of refolding of insulin glargine precursor

In order to solubilize the insulin glargine precursor manufactured by the present applicant, 3.75 g of the insulin glargine precursor was added with a 0.2 M Tris-HCl buffer solution so that the volume thereof was adjusted to 250 mL, followed by stirring at room temperature for 1 hour. The solubilized solution was diluted 10-fold with a 0.2 M Tris-HCl buffer solution containing 0.4 mM cysteine as an oxidizing agent for refolding, and was then stirred at a low temperature for 10 hours. After completion of stirring, the pH of the resulting solution was lowered to 9.0 using hydrochloric acid.

### Example 2. Selection of ion exchange resin for anion exchange chromatography purification

Impurities must be removed after refolding of the insulin precursor to increase the efficiency of enzymatic conversion of the insulin glargine precursor into insulin glargine. In order to remove impurities, anion exchange chromatography was adopted in the present invention, and a process was developed to maximize the yield by reducing insulin precursor loss during purification.

First, in order to select an ion exchange resin suitable for anion exchange chromatography, dynamic binding capacity was measured using Fractogel EMD DEAE (Merck) and Poros 50D (Thermo Fisher Scientific).

Each anion exchange resin was placed in a column having a diameter of 1 cm and a height of 20 cm. In order to allow the refolded insulin glargine precursor to sufficiently bind to the anion exchange resin, 50 mM Tris-HCl (first buffer solution), which is a buffer solution for equilibrating the anion exchange resin, was applied at various pH values, and then a 50 mM Tris-HCl buffer (second buffer solution) containing 0.5 M sodium chloride was applied at various pH values so that as much of the insulin glargine precursor was eluted as possible (Table 1).

**[Table 1]**

| Type of ion resin | First buffer solution | Second buffer solution | Dynamic binding capacity (g/L) |
|---|---|---|---|
| Fractogel EMD DEAE | 8.0 | 8.0 | ≥ 52.7 |
| | 8.5 | 8.5 | ≥ 38.1 |
| | 9.0 | 9.0 | ≥ 27.7 |
| Poros 50D | 8.0 | 8.0 | ≥ 24.1 |
| | 8.5 | 8.5 | ≥ 30.3 |
| | 9.0 | 9.0 | ≥ 34.5 |

As the results shown in Table 1, Fractogel EMD DEAE exhibited the highest dynamic binding capacity (g/L) when the first buffer solution at a pH of 8.0 and the second buffer solution at a pH of 8.0 were used, so an additional experiment was carried out using Fractogel EMD DEAE.

### Example 3. Selection of first buffer solution for Fractogel EMD DEAE

When using the Fractogel EMD DEAE ion exchange resin, an experiment was carried out while changing the pH of the first buffer solution and the concentration of Tris-HCl so as to maximize the yield of the insulin glargine precursor.

The pH of the first buffer solution was adjusted to 7.8 or 8.0 and the concentration of Tris-HCl was adjusted to 20 mM or 50 mM to equilibrate the Fractogel EMD DEAE ion exchange resin. Thereafter, a solution including the insulin glargine precursor refolded in Example 1 was added thereto so that the insulin glargine precursor was bound to the ion exchange resin, followed by elution using a 50 mM Tris-HCl buffer solution (second buffer solution) at a pH of 9.2 containing 0.5 M sodium chloride. The purity and yield of the eluted insulin glargine precursor were analyzed through RP-HPLC.

**[Table 2]**

| First buffer solution | Second buffer solution | Purity (%) | Yield (%) |
|---|---|---|---|
| 20 mM Tris-HCl, pH 7.8 | 50 mM Tris-HCl/ 0.5 M sodium chloride, pH 9.2 | 79.5 | 82.4 |
| 50 mM Tris-HCl, pH 7.8 | | 78.5 | 79.7 |
| 20 mM Tris-HCl, pH 8.0 | | 77.3 | 79.5 |
| 50 mM Tris-HCl, pH 8.0 | | 76.7 | 72.7 |

As the results shown in Table 2, when 20 mM Tris-HCl at a pH of 7.8 was used as the first buffer solution for the Fractogel EMD DEAE ion exchange resin, the yield was 82.4% and the purity was 79.5%, showing the best effect. An additional experiment was carried out under the corresponding conditions.

Example 4. Selection of second buffer solution suitable for Fractogel EMD DEAE

In addition, when using the Fractogel EMD DEAE ion exchange resin, an experiment was carried out while changing the pH of the second buffer solution and the concentration of sodium chloride that was added so as to maximize the yield of the insulin glargine precursor.

The ion exchange resin was equilibrated with the first buffer solution (20 mM Tris-HCl, pH 7.9) selected in Example 3, and a solution containing the insulin glargine precursor refolded in Example 1 was added thereto so that the insulin glargine precursor was bound to the ion exchange resin, followed by elution using a second buffer solution. Here, the second buffer solution was prepared by adjusting the pH of 50 mM Tris-HCl to 9.0 or 9.2 and adding 50 mM or 100 mM sodium chloride to 50 mM Tris-HCl at a pH of 9.0. The purity and yield of the eluted insulin glargine precursor were analyzed through RP-HPLC.

**[Table 3]**

| Second buffer solution | | Purity (%) | Yield (%) |
|---|---|---|---|
| 50 mM Tris-HCl | pH 9.0 | 23.9 | 0.0 |
| | 50 mM sodium chloride, pH 9.0 | 76.9 | 91.2 |
| | 100 mM sodium chloride, pH 9.0 | 72.7 | 93.4 |
| | pH 9.2 | 79.0 | 28.4 |

As the results shown in Table 3, when the second buffer solution for the Fractogel EMD DEAE ion exchange resin was 50 mM Tris-HCl at a pH of 9.0 containing 100 mM sodium chloride, the yield of the insulin precursor was the highest, namely 93.4%.

### Example 5. Additional selection of second buffer solution suitable for Fractogel EMD DEAE

In order to attain a second buffer solution having a further improved effect compared to the composition of the second buffer solution confirmed in Example 4, the type of buffer solution was changed from Tris-HCl to borate.

The ion exchange resin was equilibrated with the first buffer solution (20 mM Tris-HCl, pH 7.9) selected in Example 3, and a solution containing the insulin glargine precursor refolded in Example 1 was added dropwise thereto so that the insulin glargine precursor was bound to the ion exchange resin, after which the insulin glargine precursor was eluted using a second buffer solution, prepared by adjusting the pH of 50 mM borate to 9.2 or 9.4 and adding 10 mM, 30 mM or 50 mM sodium chloride thereto. The purity and yield of the eluted insulin glargine precursor were analyzed through RP-HPLC.

**[Table 4]**

| Second buffer solution | | Purity (%) | Yield (%) |
|---|---|---|---|
| 50 mM borate | 50 mM sodium chloride, pH 9.2 | 78.8 | 93.3 |
| | 30 mM sodium chloride, pH 9.2 | 80.5 | 93.5 |
| | 10 mM sodium chloride, pH 9.2 | 82.8 | 100.0 |
| | 30 mM sodium chloride, pH 9.4 | 77.8 | 100.0 |
| | 10 mM sodium chloride, pH 9.4 | 81.5 | 88.8 |

As the results shown in Table 5, when 50 mM borate at a pH of 9.4 containing 30 mM sodium chloride and 50 mM borate at a pH of 9.2 containing 10 mM sodium chloride were used, the insulin glargine precursor was purified with the highest yield.

### Example 6. Selection of first buffer solution suitable for second buffer solution borate for Fractogel EMD DEAE

In order to further improve the effect of the second buffer solution selected in Example 5, an experiment was carried out using different first buffer solution compositions.

The experiment was conducted under the same conditions as in Example 5, with the exception that 20 mM borate at a pH of 8.2 was used as the first buffer solution in lieu of 20 mM Tris-HCl at a pH of 7.9.

**[Table 5]**

| First buffer solution | Second buffer solution | Purity (%) | Yield (%) |
|---|---|---|---|
| 20 mM Tris-HCl, pH 7.9 | 50 mM borate/ 10 mM sodium chloride, pH 9.2 | 58.1 | 91.2 |
| 20 mM borate, pH 8.2 | | 54.0 | 85.7 |

As the results shown in Table 5, when 20 mM Tris-HCl at a pH of 7.9 was used, the yield of the insulin glargine precursor was higher.

### Example 7. Comparison of yield and purity depending on conditions of buffer solution for Fractogel EMD DEAE

When using the Fractogel EMD DEAE ion exchange resin, the results of yield and purity of the insulin glargine precursor were compared depending on the types and conditions of the buffer solutions.

To this end, the ion exchange resin was equilibrated under the conditions of the first buffer solution shown in Table 6 below, and a solution containing the insulin glargine precursor refolded in Example 1 was added thereto so that the insulin glargine precursor was bound to the ion exchange resin, followed by elution under the conditions of the second buffer solution shown in Table 6 below.

**[Table 6]**

| First buffer solution | Second buffer solution | Purity (%) | Yield (%) |
|---|---|---|---|
| 50 mM Tris-HCl, pH 8.0 | 50 mM Tris-HCl, pH 8.0 0.5 M sodium chloride | 69.7 | 44.7 |
| 50 mM Tris-HCl, pH 8.0 | 50 mM Tris-HCl, pH 9.2 | 77.9 | 84.2 |

As the results shown in Table 6, a higher effect was exhibited upon elution using 50 mM Tris-HCl at a pH of 9.2 than upon elution using 0.5 M sodium chloride while the pH was maintained at 8.0.

### Example 8. Selection of second buffer solution suitable for Capto DEAE

A buffer solution suitable for a Capto DEAE ion exchange resin of the same cellulose series, as the ion exchange resin that may be used in lieu of the Fractogel EMD DEAE ion exchange resin, was selected.

To this end, the ion exchange resin was equilibrated with 20 mM Tris-HCl at a pH of 7.9 as the first buffer solution, after which a solution containing the insulin glargine precursor refolded in Example 1 was introduced thereto so that the insulin glargine precursor was bound to the ion exchange resin, followed by elution using 50 mM Tris-HCl at a pH of 7.5 to 8.0 containing 100-200 mM sodium chloride.

**[Table 7]**

| Second buffer solution | | Purity (%) | Yield (%) |
|---|---|---|---|
| 50 mM Tris-HCl | 100 mM sodium chloride, pH 7.5 | 84.6 | 40.6 |
| | 150 mM sodium chloride, pH 7.5 | 78.5 | 59.8 |
| | 100 mM sodium chloride, pH 7.0 | 81.9 | 31.3 |
| | 150 mM sodium chloride, pH 8.0 | 76.5 | 64.9 |
| | 150 mM sodium chloride, pH 9.0 | 79.0 | 61.9 |
| | 200 mM sodium chloride, pH 8.0 | 78.5 | 66.9 |

As the results shown in Table 7, the strongest effect was exhibited upon elution using 50 mM Tris-HCl at a pH of 8.0 containing 200 mM sodium chloride.

Although specific embodiments of the present invention have been disclosed in detail as described above, it will be obvious to those of ordinary skill in the art that the description is merely of preferable exemplary embodiments and is not to be construed as limiting the scope of the present invention. Therefore, the substantial scope of the present invention will be defined by the appended claims and equivalents thereof.

### [Industrial Applicability]

According to the present invention, a method for purifying an insulin precursor is capable of strengthening the binding of the insulin precursor to the ion exchange resin through appropriate pH control of buffer solutions and of enabling the insulin precursor to be effectively eluted thereafter, thereby purifying an insulin precursor with high purity and high yield, and is thus very useful for high-yield insulin production through enzymatic conversion after the purification process.

### [Sequence List Free Text]

An electronic file is attached.

## Claims

1. A method for purifying an insulin precursor comprising:
(a) equilibrating an anion exchange resin with a first buffer solution;
(b) introducing a solution comprising an insulin precursor into the equilibrated anion exchange resin; and
(c) eluting the insulin precursor bound to the anion exchange resin using a second buffer solution having a pH higher than a pH of the first buffer solution.

2. The method according to claim 1, wherein the anion exchange resin is a diethylaminoethyl cellulose-based resin.

3. The method according to claim 2, wherein the anion exchange resin is Fractogel EMD DEAE or Capto DEAE.

4. The method according to claim 1, wherein the first buffer solution is 20 mM Tris-HCl at a pH of 7.0 to 8.0.

5. The method according to claim 1, wherein the second buffer solution is 10-100 mM Tris-HCl or borate at a pH of 8.0 to 10.0 containing 0-200 mM sodium chloride.

6. The method according to claim 5, wherein the second buffer solution is selected from the group consisting of:
(i) 50 mM Tris-HCl at a pH of 8.0 to 9.0 containing 0-100 mM sodium chloride (NaCl);
(ii) 50 mM borate at a pH of 9.2 containing 10 mM sodium chloride (NaCl);
(iii) 50 mM borate at a pH of 9.4 containing 30 mM sodium chloride (NaCl); and
(iv) 50 mM Tris-HCl at a pH of 8.0 to 9.0 containing 150-200 mM sodium chloride (NaCl).

7. The method according to claim 1, wherein the insulin precursor is an insulin glargine precursor.
